**Europäisches Patentamt**

(19) **European Patent Office**

**Office européen des brevets**

(11) Veröffentlichungsnummer : **0 195 198**
**B1**

(12) **EUROPÄISCHE PATENTSCHRIFT**

(45) Veröffentlichungstag der Patentschrift :
**15.03.89**

(21) Anmeldenummer : **86101004.9**

(22) Anmeldetag : **25.01.86**

(51) Int. Cl.⁴ : **C 07 C 11/04, C 07 C 9/06, C 07 C 2/84**

(54) Verfahren zur Herstellung von Ethylen-Ethan-Gemischen.

(30) Priorität : **11.03.85 DE 3508571**

(43) Veröffentlichungstag der Anmeldung :
**24.09.86 Patentblatt 86/39**

(45) Bekanntmachung des Hinweises auf die Patenterteilung : **15.03.89 Patentblatt 89/11**

(84) Benannte Vertragsstaaten :
**AT BE CH DE FR GB IT LI NL**

(56) Entgegenhaltungen :
**US--A-- 4 443 647**
**US--A-- 4 495 374**

(73) Patentinhaber : **Akzo Patente GmbH**
**Postfach 10 01 49 Kasinostrasse 19-23**
**D-5600 Wuppertal-1 (DE)**

(72) Erfinder : **Wohlfahrt, Klaus, Dr. Dipl.-Ing.**
**Römerstrasse 88**
**D-8753 Obernburg (DE)**
Erfinder : **Bergfeld, Manfred, Dr. Dipl.-Chem.**
**August-Pfeffer-Strasse 6**
**D-8765 Erlenbach-Mechenhard (DE)**
Erfinder : **Zengel, Hans, Dr. Dipl.-Ing.**
**Nordring 6**
**D-8751 Kleinwallstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 195 198 B1

**Beschreibung**

Die vorliegende Erfindung betrifft ein Verfahren zur Herstellung von Ethylen und Ethan durch oxidative Kopplung von Methan mittels Sauerstoff oder eines sauerstoffhaltigen Gases in Gegenwart eines Katalysators.

Die Herstellungsverfahren für das in der chemischen Synthese vielfältig eingesetzte Ausgangsmaterial Ethylen beruhen zur Zeit fast ausschließlich auf dem Kracken von Erdöldestillaten oder Erdgaskondensaten aus « nassem » Erdgas (Ethan und höhere Kohlenwasserstoffe). Dem technischen Krackprozeß müssen noch umfangreiche Reinigungsschritte und Gastrennverfahren nachgeschaltet werden, um das Ethylen in der für die Weiterverarbeitung erforderlichen Reinheit zu erhalten. Weniger aufwendige Aufarbeitungsschritte sind bei der Herstellung von Ethylen aus Ethan erforderlich ; doch ist die Verfügbarkeit von Ethan begrenzt.

Demgegenüber ist Methan ein in den natürlichen Lagerstätten reichlich vorhandener Rohstoff. So enthält Erdgas das Methan zu über 90 %. Es besteht daher ein Interesse an einem wirtschaftlichen Verfahren zur Herstellung von Ethylen aus Methan durch oxidative Kopplung.

Über die Herstellung von niederen Olefinen aus einfachen Ausgangsverbindungen ist vielfach berichtet worden. So beschreiben T. Inui et al. in Hydrocarbon Proc., Nov. 1982, Seite 117 ein Verfahren, bei dem ein Gemisch niederer Olefine aus Methanol erhalten wird. Y. C. Hu beschreibt in Hydrocarbon Proc., Mai 1983, Seite 88 ein Verfahren, bei dem Synthesegas durch Fischer-Tropsch-Reaktion in ein Gemisch aus Olefinen, Paraffinen und Kohlendioxid umgesetzt wird. Diese Verfahren haben insbesondere den Nachteil, daß unter Druck gearbeitet werden muß und Koksbildung festzustellen ist.

Auch ist schon die Umsetzung von Methan in Gegenwart von Sauerstoff bei Atmosphärendruck in einem Reaktionsschritt durchgeführt worden. So berichten G. E. Keller und M. M. Bhasin in J. Catal. 73 (1982), Seiten 9-19 von einem Verfahren, bei dem die Umsetzung in Gegenwart zahlreicher Metalloxide als Katalysator bei 500 bis 1 000 °C untersucht wurd und als Reaktionsprodukte neben Kohlenmonoxid und Kohlendioxid ein Gemisch von Ethylen und Ethan gebildet wurde. Diesem Verfahren haftet jedoch der Mangel einer sehr niedrigen C$_2$-Kohlenwasserstoff-Selektivität an. Zur Verbesserung der Selektivitäten schlagen diese Autoren eine allerdings apparativ sehr aufwendige und komplizierte Arbeitsweise vor.

Unter Hinweis auf die Mängel dieser Vorveröffentlichung wird nun in der DE-OS 32 37 079 ein Verfahren vorgeschlagen, das auch ohne aufwendige und komplizierte Prozeßführung zu vergleichbaren, teilweise sogar auch besseren Selektivitäten und höheren Raumzeitausbeuten an C$_2$-Kohlenwasserstoffen führt (vgl. DE-OS 32 37 079, Seite 2, letzter Absatz, bis Seite 3, Absatz 3). Gemäß dieser Druckschrift wird ein Verfahren zur Erzeugung von Ethan und/oder Ethylen beschrieben, bei dem Methan und Sauerstoff in Gegenwart eines in einer Wirbelschicht fluidisierten oder in einem Reaktor fest angeordneten Katalysators bei Temperaturen zwischen 500 und 900 °C in einem bestimmten Bereich der Partialdruck-Verhältnisse von Methan und Sauerstoff umgesetzt werden. Der Katalysator soll ein Oxid mehrwertiger Metalle sein (vgl. DE-OS 32 37 079, Seite 3, letzter Absatz). Als bevorzugte Katalysatoren werden Produkte mit Oxiden des Bleis, Antimons, Zinns, Wismuts, Cadmiums, Thalliums und Indiums oder deren Mischungen als aktive Komponente genannt, als besonders bevorzugt jedoch Bleioxid oder seine Mischung mit Antimonoxid. Die Metalloxide können als solche oder fein verteilt auf der Oberfläche eines Trägermaterials wie Aluminiumoxid oder Siliciumdioxid zum Einsatz kommen (vgl. Anspruch 6 sowie Seite 4, Absatz 4). Wie die Ergebnisse der Ausführungsbeispiele in Tabelle 1 nun zeigen, wird jedoch auch mit dem besonders bevorzugten Bleioxid (auf einem Träger) eine Selektivität für Kohlenwasserstoffe von maximal nur ca. 52,9 % (Methan-Umsatz 7 %) erreicht. — Über weitergehende Arbeiten zur Erhöhung der Sekektivität haben W. Hinsen, W. Bytyn und M. Baerns auf dem 8. Internationalen Kongreß der Katalyse 1984 in Berlin berichtet. Gemäß Kongreß-Buch « 8th International Congress on Catalysis », Vol. III, Seiten 581-592, Verlag Chemie, Weinheim 1984 kann die Kohlenwasserstoff-Selektivität des Bleioxid-Katalysators bei geeigneter Auswahl des Trägermaterials und durch Alkalizusatz erhöht werden. Von den untersuchten Trägermaterialien α-Aluminiumoxid, γ-Aluminiumoxid, Titandioxid, Aluminiumsilikat, Silikagel erwies sich dabei γ-Aluminiumoxid am geeignetsten ; es führte zu Selektivitäten von maximal 57,7 % (Methan-Umsatz 7,1 %). Für eine kommerzielle Nutzung des Verfahrens der oxidativen Kopplung von Methan besteht jedoch das Bedürfnis nach einer wesentlichen Erhöhung der Selektivität und des Methan-Umsatzes.

Aufgabe der vorliegenden Erfindung ist es daher, Methan nach dem im grundlegenden Prinzip bekannten Verfahren der oxidativen Kopplung in einer katalysierten Reaktion mit höherer Selektivität bzw. brauchbaren Methan-Umsätzen insbesondere zu C$_2$-Kohlenwasserstoffen umzusetzen.

Diese Aufgabe wird gelöst durch ein Verfahren zur Herstellung von Ethan und Ethylen durch Oxidation von Methan mittels molekularem Sauerstoff oder eines sauerstoffhaltigen Gases in Gegenwart von Blei-II-oxid als Katalysator bei einer Temperatur von 600 bis 900 °C, das dadurch gekennzeichnet ist, daß man das Blei-II-oxid

auf einem Träger verteilt einsetzt, der aus Bimsstein, Siliciumcarbid, Zinkoxid, Zirkonoxid und/oder aus Oxiden der Erdalkalielemente, gegebenenfalls im Gemisch mit Natriumsilikat besteht, oder

im Gemisch mit Mangan-II-oxid auf einem Träger verteilt einsetzt, der aus Bimsstein, Siliciumcarbid, Siliciumdioxid, Titandioxid, Zirkondioxid, Mangan-II-oxid, Zinkoxid und/oder Oxiden der Erdalkalielemente, gegebenenfalls im Gemisch mit Natriumsilikat, besteht, oder

ohne Träger im Gemisch mit Natriumsilikat allein oder zusammen mit Siliciumdioxid, Titandioxid, Zirkondioxid, Zinkoxid und/oder Oxiden der Erdalkalielemente einsetzt.

Erfindungsgemäß werden in allen Ausführungsformen als Oxide der Erdalkalielemente die Oxide von Magnesium und Calcium bevorzugt.

In derjenigen erfindungsgemäßen Ausführungsform, in welcher Blei-II-oxid im Gemisch mit Mangan-II-oxid als Katalysator eingesetzt wird, werden Gemische von Blei-II-oxid und Mangan-II-oxid im Molverhältnis 1 : 0,1 bis 1 : 3 bevorzugt. Besonders vorteilhaft sind Gemische von Blei-II-oxid und Mangan-II-oxid im Molverhältnis 1 : 0,2 bis 1 : 0,5 oder 1 : 1,5 bis 1 : 2.

In den erfindungsgemäßen Ausführungsformen, in denen die Katalysatoren auf einem Träger eingesetzt werden, wählt man bevorzugt einen Anteil von 0,004 bis 0,300 Mol Katalysator auf 100 ml Träger. Besonders vorteilhaft werden 0,012 bis 0,190 Mol Katalysator auf 100 ml Träger eingesetzt.

In der erfindungsgemäßen Ausführungsform, in welcher der Katalysator ohne Träger eingesetzt wird und aus Blei-II-oxid im Gemisch mit Wasserglas und Siliciumdioxid, Titandioxid, Zirkondioxid, Zinkoxid und/oder Oxiden der Erdalkalielemente besteht, sind Gemische mit Blei-II-oxid und diesen anderen Oxiden im Molverhältnis von 1 : 4 bis 1 : 40 besonders geeignet.

Sämtliche erfindungsgemäßen Katalysatoren können sowohl im Fließbett als auch im Festbett angeordnet werden.

Die erfindungsgemäßen Katalysatoren können wie folgt hergestellt werden :

Für den Einsatz der Katalysatoren auf einem Träger werden entsprechende lösliche Verbindungen der betreffenden Metalle, welche bei höheren Temperaturen die Oxide freisetzen, in einem geeigneten Lösungsmittel, zum Beispiel Nitrate in Wasser, gelöst und das Trägermaterial in Pelletform in der resultierenden Lösung suspendiert. Die Lösung mit suspendiertem Träger wird im Vakuum zur Trockne eingeengt und bei ca. 100 °C im Vakuum getrocknet.

Für den Einsatz ohne Träger werden erfindungsgemäß die Katalysatoren zusammen mit einem Bindemittel eingesetzt. In diesem Falle werden die jeweiligen Komponenten (das Oxid des jeweiligen Metalls freisetzende Verbindungen) zum Beispiel in einer Reibschale mit Natronwasserglas angeteigt und gut durchgeknetet. Anschließend wird etwas konzentrierte Ammoniaklösung zugefügt und durchgeknetet, um die Polymerisation des Wasserglases zum Katalysator-Bindemittel einzuleiten, wobei die Masse langsam erstarrt. Mit dieser Masse wird eine Metallplatte beschichtet (Schichthöhe ca. 5 mm). Nach dem Trocknen dieser Schicht im Vakuum bei ca. 80 °C werden die hierbei entstandenen spröden Katalysator-Partikel noch bei ca. 600 °C kalziniert und dann auf die gewünschte Korngröße zerkleinert.

Auch die erfindungsgemäß einzusetzenden Träger können das Polymerisationsprodukt von Wasserglas erforderlichenfalls als Bindemittel enthalten.

Die nachstehend beschriebenen Versuche wurden in einer Quarzröhre mit einem inneren Durchmesser von 11,3 mm durchgeführt. Nach Einfüllen des Katalysators in dieser Röhre wird dieser im Stickstoffstrom auf Reaktionstemperatur gebracht. Die Röhre wird auf einer Länge von 0,5 m mittels eines Strahlungsrohrofens erhitzt. Methan und Sauerstoff oder Luft werden über Strömungsmesser in diesen Reaktor geführt und über den jeweiligen Katalysator gleitet. Das austretende Reaktionsgemisch wird auf einer Temperatur oberhalb 80 °C gehalten und direkt zur Bestimmung im Gaschromatographen geleitet. — Dieses Gemisch enthält als Folgeprodukte des Oxidationsvorgangs Kohlenmonoxid, Kohlenoxid, Wasser, Ethylen, Ethan und in geringeren Mengen höhere Kohlenwasserstoffe, insbesondere $C_3$- und $C_4$-Kohlenwasserstoffe. Rußbildung ist nicht festzustellen.

Die einzelnen Verfahrensbedingungen und Ergebnisse sind in den Tabellen 1-5 zusammengefaßt.

Von wesentlichem Einfluß auf Selektivität und Umsatz ist das Verhältnis von Methan und Sauerstoff im eingespeisten Gasgemisch, wie aus Tabelle 1 ersichtlich ist.

Tabelle 1

60 g eines Katalysators, hergestellt aus 13,4 g PbO, 21,2 g Wasserglas, 42 g ZnO, 0,4 ml konz. Ammoniak, mit $HNO_3$ auf $p_H = 4$ eingestellt, 750 °C
kein Träger

| Zuleitung (l/h) | | Umsatz (%) | | Selektivität (%) | | | | | |
|---|---|---|---|---|---|---|---|---|---|
| Methan | Luft (25 °C) | $CH_4$ | $O_2$ | $CO_2$ + CO | $C_2H_4$ | $C_2H_6$ | $C_3$ | $C_4$ | $\sum$ KW |
| 66 | 34 | 7,43 | | 25,7 | 35,6 | 35,2 | 3,4 | 0 | 74,2 |
| 50 | 50 | 13,31 | 62,3 | 37,6 | 32,1 | 24,1 | 3,1 | 2,9 | 62,2 |
| 34 | 66 | 20,08 | 59,2 | 48,5 | 30,9 | 18,1 | 2,5 | 0 | 51,5 |
| | Sauerstoff | | | | | | | | |
| 90 | 10 | 8,09 | 71 | 27,9 | 34,3 | 33,8 | 3,9 | 0 | 72,0 |
| 80 | 20 | 17,70 | 73 | 39,9 | 29,0 | 20,7 | 3,2 | 7,1 | 60,0 |
| 66,6 | 33,3 | 26,34 | 61 | 51,0 | 25,3 | 15,5 | 2,6 | 5,4 | 48,8 |

Durch eine Erniedrigung des Sauerstoffanteils kann also die Selektivität verbessert werden, während durch Erhöhung des Sauerstoffanteils der Methanumsatz erhöht wird.

Eine andere Möglichkeit zur weiteren Verbesserung der Selektivität bei gleichzeitiger Erhöhung des Methanumsatzes ist eine Verfahrensweise mit Seiteneinspeisung des Sauerstoffs. Hierbei wird am einen Ende der Reaktorröhre nur das Methan zugeführt, während die Gesamtmenge des Sauerstoffs auf mehrere seitlich angebrachte Eintrittsöffnungen verteilt in die Röhre eingespeist wird.

Das erfindungsgemäße Verfahren kann bei Temperaturen im Bereich zwischen 600 und 900 °C durchgeführt werden. Bevorzugt wird ein Temperaturbereich zwischen 650 und 850 °C.

Der Einfluß der Temperatur auf die oxidative Kopplung des Methans mit einem erfindungsgemäßen Katalysator wird in Tabelle 2 veranschaulicht.

Tabelle 2

45 g des gemäß Tabelle 1 verwendeten Katalysators
33 l/h $CH_4$, 66 l/h Luft (bei Raumtemperatur)

| T. (°C) | $O_2$-Umsatz (%) | $CH_4$-Umsatz (%) | Selektivität (%) | | | | | |
|---|---|---|---|---|---|---|---|---|
| | | | $CO + CO_2$ | $C_2H_4$ | $C_2H_6$ | $C_3$ | $C_4$ | $\sum$KW |
| 650 | 4 | 1,1 | 37,3 | 20,9 | 41,8 | 0 | 0 | 62,7 |
| 700 | 18 | 3,3 | 25,3 | 24,4 | 50,5 | 0 | 0 | 74,9 |
| 750 | 95 | 12,3 | 25,4 | 36,3 | 32,0 | 4,0 | 2,3 | 74,6 |
| 800 | 95 | 13,6 | 27,2 | 41,3 | 28,0 | 3,9 | 1,7 | 74,9 |
| 850 | 95 | 12,5 | 27,8 | 43,9 | 21,0 | 5,0 | 2,3 | 72,2 |

Es zeigt sich also, daß mit höherer Temperatur höhere Anteile an Ethylen gebildet werden und der Anteil an Ethan sich entsprechend verringert ; die Summe aus Ethylen und Ethan bleibt fast konstant.

Von Einfluß auf die Selektivität ist auch der $p_H$-Wert während der Zubereitung des Katalysators. In Tabelle 3 sind die Resultate von erfindungsgemäßen Katalysatoren wiedergegeben, denen bei der Zubereitung bestimmte Mengen Calciumoxid bzw. Salpetersäure zur Einstellung des jeweiligen $p_H$-Werts zugesetzt wurden.

Tabelle 3

45 g des gemäß Tabelle 1 verwendeten Katalysators $p_H$-Wert zunächst 12 ; Einstellen des jeweiligen $p_H$-Wertes durch CaO- bzw. $HNO_3$-Zugabe
33 l/h $CH_4$, 66 l/h Luft (bei Raumtemperatur)
750 °C

| $p_H$ | Umsatz (%) | | $C_2$-KW-Selektivität (%) |
|---|---|---|---|
| | $CH_4$ | $O_2$ | |
| 12,5 | 10,8 | 95 | 58 |
| 9 | 11,1 | 95 | 64 |
| 6,5 | 12,3 | 95 | 67 |
| 4 | 13,4 | 95 | 70 |
| 3 | 9,1 | 81 | 64 |

In den nachfolgenden Tabellen 4 und 5 sind die Resultate von Versuchen mit weiteren zahlreichen erfindungsgemäßen Katalysatoren bzw. Katalysator-Träger-Kombinationen zusammengestellt.

(Siehe Tabelle 4 Seite 5 f.)

Tabelle 4

ca. 50 ml Katalysator (Aktivkomponente + Träger)
0,06 Mol Aktivkomponente auf 60 ml Träger
66 l Methan/h
33 l Luft (25 °C)/h
750 °C

| Vers. Nr. | Katalysator * (Aktivkomponente) | Träger | Umsatz $CH_4$ (%) | $C_2$-KW-Selektivität (%) |
|---|---|---|---|---|
| 1 | PbO | Zinkoxid | 11,8 | 69 |
| 2 | PbO | Magnesiumoxid | 10,8 | 59 |
| 3 | PbO | Calciumoxid | 10,6 | 60 |
| 4 | PbO | Zirkonoxid | 11,8 | 60 |
| 5 | PbO | Wasserglas-gebundenes Zinkoxid | 10,2 | 73 |
| 6 | PbO | Silicium-carbid | 9,6 | 69 |
| 7 | PbO | Bimsstein | 0,6 | 95 |
| 8 | PbO : MnO (1:2) | Kieselgel ** | 9,1 | 58 |
| 9 | PbO : MnO (5:1) | Kieselgel ** | 12,6 | 70,5 (mit $C_3$-KW:73,7) |

\* Zahlenangaben in Klammern : Molverhältnisse
\*\* Kieselgel EM der Fa. Gebr. Herrmann, Köln-Ehrenfeld

Tabelle 5

ca. 45 ml eines mit Wasserglas zubereiteten Katalysators     66 l Methan/h
kein Träger     33 l Luft (25 °C)/h
750 °C

| Vers. Nr. | Katalysator * | pH-Wert bei Katalysator-zubereitung | Umsatz $CH_4$ (%) | KW-Selektivität (%) | | | | |
|---|---|---|---|---|---|---|---|---|
| | | | | $C_2H_4$ | $C_2H_6$ | $C_3H_8$ | $C_4H_{10}$ | $\sum$KW |
| 1 | PbO | 12 | 0,3 | 66 | 0 | 0 | 0 | 66 |
| 2 | PbO : ZnO (1:8) | 4 | 12,3 | 36,3 | 32,0 | 4,0 | 2,3 | 74,6 |
| 3 | PbO : ZnO (1:40) | 4 | 11,4 | 25,5 | 37,7 | 8,2 | 0 | 63,2 |
| 4 | PbO : ZnO : $ZrO_2$ (1:4:3) | 12 | 4,3 | 17,0 | 60,5 | 0 | 0 | 77,5 |
| 5 | PbO : $TiO_2$ (1:8) | 4 | 6,3 | 24,9 | 46,9 | 3,6 | 0 | 75,4 |
| 6 | PbO : $ZrO_2$ (1:4) | 4 | 10,2 | 31,8 | 33,4 | 3,7 | 0 | 68,9 |
| 7 | PbO : MgO (1:8) | 12 | 9,4 | 12,0 | 40,0 | 1,4 | 0 | 53,4 |
| 8 | PbO : CaO (1:8) | 7 | 10,9 | 19,0 | 45,3 | 0 | 0 | 64,3 |
| 9 | PbO : MnO (1:2) | 12 | 10,6 | 21.9 | 40,4 | 3,0 | 0 | 65,3 |
| 10 | PbO : MnO : ZnO (3:1:25) | 4 | 12,2 | 23,3 | 42,8 | 2,9 | 0 | 69,0 |

\* Zahlen-Angaben in Klammern : Molverhältnisse

Den Tabellen 1, 2 und 5 kann leicht entnommen werden, welche Reaktionsbedingungen für bestimmte Zielsetzungen hinsichtlich der Endprodukt-Zusammensetzung besonders geeignet sind. Für Versuch 9 der Tabelle 4 wurde folgende Endprodukt-Zusammensetzung (KW-Selektivitäten) ermittelt: 32,5 % $C_2H_4$ ; 38,0 % $C_2H_6$ ; 3,2 % $C_3H_8$.

So kann durch entsprechende Wahl des Methan : Sauerstoff (bzw. Luft)-Verhältnisses, der Temperatur und des Katalysators ein bevorzugtes Verhältnis Ethylen : Ethan erhalten oder durch geeignete Wahl des Katalysators, wenn gewünscht, der Anteil an Kohlenwasserstoffen mit mehr als zwei Kohlenstoffatomen zurückgedrängt werden.

In vielen Fällen werden efindungsgemäß sehr hohe Kohlenwasserstoff-Selektivitäten > 70 % bei brauchbaren Aktivitäten des Katalysators erhalten. In anderen Fällen resultiert sogar eine Selektivität > 90 % bei allerdings niederen Aktivitäten. Hinsichtlich der Bleioxidkatalysatoren auf Träger ist festzustellen, daß es je nach verwendetem Träger zu einer hohen Selektivität bei guter Aktivität (zum Beispiel Zinkoxid als Träger) oder zu einer sehr hohen Selektivität bei geringer Aktivität führen kann (zum Beispiel Bimsstein als Träger).

Das erfindungsgemäße Verfahren ist somit den bisher beschriebenen Verfahren überlegen und für eine witschaftliche Herstellung von Ethan und Ethylen durch oxidative Kopplung von Methan geeignet.

Hierfür empfiehlt sich eine Prozeßführung mit niedrigem Sauerstoff-Anteil im Reaktor, wie sie zum Beispiel mit einem Fließbettreaktor, Kreislaufreaktor, Seiteneinspeisung oder anderen Maßnahmen der Konzentrationsführung erreicht werden kann. — Das Reaktionsgemisch kann bei technischer Verfahrensweise mittels konventioneller Raffineriegastechnologie getrennt oder gegebenenfalls ohne Trennung zum Beispiel zu chlorierten Produkten umgesetzt werden.

**Patentansprüche**

1. Verfahren zur Herstellung von Ethan und Ethylen durch Oxidation von Methan mittels molekularem Sauerstoff oder eines sauerstoffhaltigen Gases in Gegenwart von Blei-II-oxid als Katalysator bei einer Temperatur von 600-900 °C, dadurch gekennzeichnet, daß man das Blei-II-oxid

auf einem Träger verteilt einsetzt, der aus Bimsstein, Siliciumcarbid, Zinkoxid, Zirkondioxid und/oder aus Oxiden der Erdalkalielemente, gegebenenfalls im Gemisch mit Natriumsilikat, besteht, oder

im Gemisch mit Mangan-II-oxid auf einem Träger verteilt einsetzt, der aus Bimsstein, Siliciumcarbid, Siliciumdioxid, Titandioxid, Zirkondioxid, Zinkoxid und/oder Oxiden der Erdalkalielemente, gegebenenfalls im Gemisch mit Natriumsilikat, besteht, oder

ohne Träger im Gemisch mit Natriumsilikat allein oder zusammen mit Siliciumdioxid, Titandioxid, Zirkondioxid, Mangan-II-oxid, Zinkoxid und/oder Oxiden der Erdalkalielemente einsetzt.

2. Verfahren nach Anspruch 1, dadurch gekennzeichnet, daß man die Oxidation des Methans bei 650 bis 850 °C durchführt.

3. Verfahren nach den Ansprüchen 1 und 2, dadurch gekennzeichnet, daß man als Oxide der Erdalkalielemente die Oxide von Magnesium oder Calcium einsetzt.

4. Verfahren nach den Ansprüchen 1 bis 3, dadurch gekennzeichnet, daß man als Katalysator ein Gemisch von Blei-II-oxid und Mangan-II-oxid im Molverhältnis 1 : 0,1 bis 1 : 3 einsetzt.

5. Verfahren nach den Ansprüchen 1 bis 4, dadurch gekennzeichnet, daß man als Katalysator ein Gemisch von Blei-II-oxid und Mangan-II-oxid im Molverhältnis 1 : 0,2 bis 1 : 0,5 oder 1 : 1,5 bis 1 : 2 einsetzt.

6. Verfahren nach den Ansprüchen 1 bis 5, dadurch gekennzeichnet, daß 0,004 bis 0,300 Mol Katalysator auf 100 ml Träger eingesetzt werden.

7. Verfahren nach den Ansprüchen 1 bis 5, dadurch gekennzeichnet, daß 0,012 bis 0,190 Mol Katalysator auf 100 ml Träger eingesetzt werden.

8. Verfahren nach den Ansprüchen 1 bis 3 ohne Träger, dadurch gekennzeichnet, daß man Gemische von Blei-II-oxid mit Siliciumdioxid, Titandioxid, Zirkondioxid, Zinkoxid und/oder Oxiden der Erdalkalielemente im Molverhältnis 1 : 4 bis 1 : 40 einsetzt.

**Claims**

1. A process for the production of ethane and ethylene by oxidation of methane with molecular oxygen or an oxygen-containing gas at a temperature of 600 to 900 °C in the presence of lead (II) oxide as catalyst, characterized in that the lead (II) oxide used is distributed over a support consisting of pumice, silicon carbide, zinc oxide, zirconium dioxide and/or of oxides of the alkaline-earth elements, optionally in admixture with sodium silicate,

or is distributed in admixture with manganese (II) oxide over a support consisting of pumice, silicon carbide, silicon dioxide, titanium dioxide, zirconium dioxide, zinc oxide and/or oxides of the alkaline-earth elements, optionally in admixture with sodium silicate,

or is used without a support in admixture with sodium silicate alone or together with silicon dioxide, titanium di-oxide, zirconium dioxide, manganese (II) oxide, zinc oxide and/or oxides of the alkaline-earth elements.

2. A process as claimed in claim 1, characterized in that oxidation of the methane is carried out at 650 to 850 °C.

3. A process as claimed in claims 1 and 2, characterized in that the oxides of magnesium or calcium are used as oxides of the alkaline-earth elements.

4. A process as claimed in claims 1 to 3, characterized in that a mixture of lead(II) oxide and manganese(II) oxide in a molar ratio of 1 : 0.1 to 1 : 3 is used as the catalyst.

5. A process as claimed in claims 1 to 4, characterized in that a mixture of lead(II) oxide and manganese(II) oxide in a molar ratio of 1 : 0.2 to 1 : 0.5 or 1 : 1.5 to 1 : 2 is used as the catalyst.

6. A process as claimed in claims 1 to 5, characterized in that 0.004 to 0.300 mol catalyst is used to 100 ml support.

7. A process as claimed in claims 1 to 5, characterized in that 0.012 to 0.190 mol catalyst is used to 100 ml support.

8. A process as claimed in claims 1 to 3 with no support, characterized in that mixtures of lead(II) oxide with silicon dioxide, titanium dioxide, zirconium dioxide, zinc oxide and/or oxides of the alkaline-earth elements in a molar ratio of 1 : 4 to 1 : 40 are used.

**Revendications**

1. Procédé de production d'éthane et d'éthylène par oxydation du méthane au moyen d'oxygène moléculaire ou d'un gaz contenant de l'oxygène, en présence d'oxyde de plomb II comme catalyseur, à une température de 600 à 900 °C, lequel procédé est caractérisé en ce que l'on utilise l'oxyde de plomb II à l'état finement divisé sur un support, constitué de pierre ponce, carbure de silicium, oxyde de zinc, dioxyde de zirconium et/ou d'oxydes d'éléments alcalino-terreux, éventuellement en mélange avec du silicate de sodium, ou bien

mélangé à de l'oxyde de manganèse II et à l'état finement divisé sur un support, constitué de pierre ponce, carbure de silicium, dioxyde de silicium, dioxyde de titane, dioxyde de zirconium, oxyde de zinc et/ou oxydes d'éléments alcalino-terreux, éventuellement en mélange avec du silicate de sodium, ou bien

sans support, en mélange avec du silicate de sodium seul ou conjointement avec du dioxyde de silicium, du dioxyde de titane, du dioxyde de zirconium, de l'oxyde de manganèse II, de l'oxyde de zinc et/ou des oxydes d'éléments alcalino-terreux.

2. Procédé selon la revendication 1, caractérisé en ce que l'on effectue l'oxydation du méthane à une température de 650 à 850 °C.

3. Procédé selon les revendications 1 et 2, caractérisé en ce que l'on utilise, comme oxydes d'éléments alcalino-terreux, les oxydes de magnésium ou de calcium.

4. Procédé selon les revendications 1 à 3, caractérisé en ce que l'on utilise, comme catalyseur, un mélange d'oxyde de plomb II et d'oxyde de manganèse II, en un rapport molaire de 1 : 0,1 à 1 : 3.

5. Procédé selon les revendications 1 à 4, caractérisé en ce que l'on utilise, comme catalyseur, un mélange d'oxyde de plomb II et d'oxyde de manganèse II, en un rapport molaire de 1 : 0,2 à 1 : 0,5 ou bien de 1 : 1,5 à 1 : 2.

6. Procédé selon les revendications 1 à 5, caractérisé en ce que l'on utilise de 0,004 à 0,300 mole de catalyseur pour 100 ml de support.

7. Procédé selon les revendications 1 à 5, caractérisé en ce que l'on utilise de 0,012 à 0,190 mole de catalyseur pour 100 ml de support.

8. Procédé selon les revendications 1 à 3, sans support, caractérisé en ce que l'on utilise un mélange d'oxyde de plomb II avec du dioxyde de silicium, du dioxyde de titane, du dioxyde de zirconium, de l'oxyde de zinc et/ou des oxydes d'éléments alcalino-terreux, en un rapport molaire de 1 : 4 à 1 : 40.